# EUROPEAN PATENT APPLICATION

(11) **EP 4 403 910 A1**
(43) Date of publication of application: **24.07.2024**
(21) Application number: 22869685.2
(22) Date of filing: 14.07.2022
(51) Int. Cl.: G01N 27/30, G01N 33/483

(54) **ELECTROCHEMICAL SENSOR**

(30) Priority: 14.09.2021 JP 2021149638
(71) Applicant: Sumitomo Chemical Company, Limited, Tokyo 103-6020 (JP)
(72) Inventor: KURIHARA, Kaori, Hitachi-shi, Ibaraki 319-1418 (JP); OTOKI, Yohei, Hitachi-shi, Ibaraki 319-1418 (JP)
(74) Representative: Vossius & Partner Patentanwälte Rechtsanwälte mbB
(86) International application number: PCT/JP2022/027685
(87) International publication number: WO 2023/042537

(57) **Abstract**

This disclosure involves a substrate, an electrode chip including a base material and a diamond thin film, and an insulating resin configured to seal at least part of a surface of a conductive member included in the electrode chip. The insulating resin is provided so as to expose at least part of the diamond thin film. When an exposed surface of the diamond thin film is measured by time-of-flight secondary ion mass spectrometry, an integrated value B_{L} per unit area of ionic strength of organic matter with a molecular weight of 100 or more is 1.2 × 10⁶ cts/sec or less.

## Description

### TECHNICAL FIELD

The present disclosure relates to an electrochemical sensor.

### BACKGROUND ART

Recently, it has been proposed to use an electrode including a diamond thin film (also referred to as "diamond electrode" below) as a working electrode for an electrochemical sensor (see, for example, Patent Documents 1 and 2). Since conductive diamond has a wide potential window and a small background current, it enables electrochemical detection of various matters such as uric acid with high sensitivity. Therefore, conductive diamond is attracting attention as a material for forming working electrodes. Such an electrochemical sensor includes a substrate, electric wires provided on the substrate, and a diamond electrode connected to the electric wires and secured onto the substrate using, for example, a conductive paste. In the diamond electrode, a surface of a conductive member other than a diamond thin film is sealed with an insulating resin.

### PRIOR ART DOCUMENT

### PATENT DOCUMENT

Patent Document 1: Japanese Patent Laid-Open Publication No. 2007-292717
Patent Document 2: Japanese Patent Laid-Open Publication No. 2013-208259

### SUMMARY

### PROBLEMS TO BE SOLVED BY THE DISCLOSURE

With an electrochemical sensor equipped with a diamond electrode, despite the resin sealing described above being applied, the accuracy of measuring the concentration of a detection target may decrease depending on the type of test liquid. An objective of the present disclosure is to provide a diamond electrode-equipped electrochemical sensor capable of accurately measuring the concentration of a detection target regardless of the type of test liquid.

### MEANS FOR SOLVING PROBLEM

According to an aspect of the present disclosure, there is provided an electrochemical sensor including:
a substrate;
an electrode chip including a base material and a diamond thin film; and
an insulating resin configured to seal at least part of a surface of a conductive member included in the electrode chip,
wherein the insulating resin is provided so as to expose at least part of the diamond thin film, and
when an exposed surface of the diamond thin film is measured by time-of-flight secondary ion mass spectrometry, an integrated value B_{L} per unit area of ionic strength of organic matter with a molecular weight of 100 or more is 1.2 × 10⁶ cts/sec or less.

### ADVANTAGE OF DISCLOSURE

According to the present disclosure, a diamond electrode-equipped electrochemical sensor capable of accurately measuring the concentration of a detection target can be provided.

### BRIEF DESCRIPTION OF DRAWINGS

FIG. 1 is a schematic perspective view illustrating an electrochemical sensor according to an embodiment of the present disclosure.
FIG. 2 is a cross-sectional view, taken along line A-A, of the electrochemical sensor illustrated in FIG. 1.
FIG. 3 is a schematic view of a vapor phase deposition apparatus used to grow a diamond crystal.
FIG. 4(a) is a view illustrating a cross-sectional structure of a stack (stacked wafer) including a diamond thin film and a base material, FIG. 4(b) is a cross-sectional view illustrating a state where concave grooves have been formed on a rear surface of the stacked wafer illustrated in FIG. 4(a), and FIG. 4(c) is a schematic view illustrating how the base material is broken along the concave grooves to obtain an electrode chip.
FIG. 5(a) is a view illustrating a modified example of the electrochemical sensor according to an embodiment of the present disclosure, FIG. 5(b) is a view illustrating another modified example of the electrochemical sensor according to an embodiment of the present disclosure, and FIG. 5(c) is a view illustrating a yet another modified example of the electrochemical sensor according to an embodiment of the present disclosure.
FIG. 6 is a view illustrating TOF-SIMS (positive ions) results for Samples 1 and 8.
FIG. 7 is a view illustrating TOF-SIMS (negative ions) results for Samples 1 and 8.
FIG. 8 is a view illustrating evaluation results for Samples 1 to 3.
FIG. 9 is a view illustrating evaluation results for Samples 4 to 6.
FIG. 10 is a view illustrating evaluation results for Samples 7 and 8.

### DETAILED DESCRIPTION

### <Findings by the Present Inventors>

In order to measure the concentration of a detection target (e.g. uric acid) in a test liquid (e.g. human urine) collected from a living body, an electrochemical sensor equipped with an electrode chip (chip-shaped electrode) having a diamond thin film may be used. Such an electrochemical sensor includes a substrate, electric wires provided on the substrate, and an electrode chip connected to the electric wires and secured onto the substrate using a conductive bonding member such as a conductive paste. With electrochemical sensors, when a surface of a conductive member other than the diamond thin film in the electrode chip is exposed, it is difficult to accurately measure the concentration of the detection target; thus, the surface of the conductive member other than the diamond thin film needs to be sealed with an insulating resin. Resin sealing is generally performed by: applying an insulating resin in liquid form so as to cover the surface of the conductive member other than the diamond thin film; and then curing the resin by heating or ultraviolet irradiation. However, it has been found that the accuracy of measuring the concentration of a detection target may decrease despite such resin sealing being applied. This is a new problem found by the present inventors.

The present inventors have conducted extensive research on a factor of the decrease in measurement accuracy. As a result, it has been found that when the resin is cured by heating or ultraviolet irradiation, the heat at the time of heat curing or heat generated by an ultraviolet curing device may result in volatilization of organic matters with various molecular weights from the resin; these matters adhere to the diamond thin film surface, causing a decrease in measurement accuracy. Through further research, it has also been found that measurement accuracy may decrease when organic matter (volatile organic matter) having volatilized from the resin has adhered to the diamond thin film surface and the test liquid contains protein. This is because in this case, the protein may react with the volatile organic matter so that a membrane (protein membrane) may form on the diamond thin film surface and this membrane becomes an obstruction in the detection target reaching the diamond thin film surface, resulting in a decrease in measurement accuracy.

The present inventors also found that even when the volatile matter adheres to the diamond thin film surface, if the test liquid does not contain protein, the membrane does not form and accurate concentration measurement can be performed. The present inventors also found that even when the volatile matter adheres to the diamond thin film surface, if the adherence amount of the organic matter with a molecular weight of 100 or more is a certain amount or less, the membrane does not form despite the test liquid containing protein, and accurate concentration measurement can be performed.

The present disclosure has been arrived at on the basis of the problems and findings found by the present inventors as described above.

### <Embodiments of Present Disclosure>

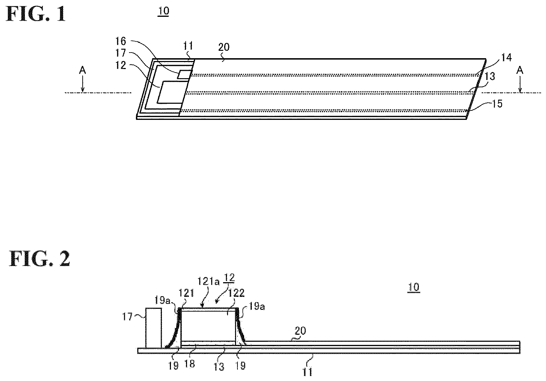

With reference to FIG. 1 and FIG. 2, an explanation will be given for an electrochemical sensor, which uses a three-electrode method to measure the concentration of a detection target in a test liquid containing protein, as an embodiment of the present disclosure. For this embodiment, the explanation will be given assuming an example in which the test liquid containing protein is human urine and the detection target is uric acid.

### <1> Configuration of Electrochemical Sensor

As illustrated in FIG. 1, an electrochemical sensor 10 (also referred to as "sensor 10" below) according to this embodiment is configured to include a substrate 11 and an electrode chip (chip-shaped electrode) 12.

The substrate 11 is formed as a sheet-like (plate-like) member. The substrate 11 can be formed of insulating materials such as insulative composite resin, ceramic, glass, plastic, flammable materials, biodegradable materials, non-woven fabric, or paper. It is preferable that the substrate 11 be formed of, for example, glass epoxy resin or polyethylene terephthalate (PET). The substrate 11 may be a semiconductor substrate or a metal substrate configured so that the surface thereof on which the electrode chip 12, etc., is to be provided has an insulating property. The planar shape of the substrate 11 may be, for example, rectangular. The substrate 11 has a predetermined physical strength and mechanical strength such that, for example, the substrate 11 will not bend or break within a certain amount of time after adherence of urine.

On one of two main surfaces of the substrate 11 (also referred to as "top surface of substrate 11" below), three wires (electrical wires) 13, 14, and 15 are provided from one end toward the other end of the substrate 11 in the longitudinal direction of the substrate 11. The three wires 13 to 15 are kept apart from each other. Examples of materials for forming the wires 13 to 15 include: noble metals such as copper (Cu), gold (Au), platinum (Pt), silver (Ag), or palladium (Pd); metals such as aluminum (Al), iron (Fe), nickel (Ni), chromium (Cr), or titanium (Ti); an alloy composed mainly of such noble metals or metals; an oxide of such noble metals or alloy; metal oxides; carbon; etc. All of the wires 13 to 15 may be formed of the same material, or each wire may be formed of a different material. The wires 13 to 15 can be formed by, for example, a subtractive method or a semi-additive method. Alternatively, the wires 13 to 15 can be formed by, for example, a printing method or an evaporation method. Exemplary printing methods include a screen printing method, a gravure printing method, an offset printing method, and an inkjet printing method.

To one end of the wire 13, the electrode chip 12 serving as a working electrode is connected through a conductive bonding member 18 (see FIG. 2). Details of the electrode chip 12 will be described later.

To one end of the wire 14, a reference electrode 16 is connected. The reference electrode 16 is an electrode used as a basis for determining a potential of the electrode chip 12 (working electrode). The reference electrode 16 can be, for example, a silver/silver chloride (Ag/AgCl) electrode. Alternatively, the reference electrode 16 can be, for example, a standard hydrogen electrode, a reversible hydrogen electrode, a palladium-hydrogen electrode, a saturated calomel electrode, or a carbon electrode. The reference electrode 16 can be connected to the wire 14 through a conductive bonding member such as a conductive paste. The reference electrode 16 can also be an electrode formed of, for example, a metal such as Pt, Au, Cu, Pd, Ni, or Ag. In this case, the reference electrode 16 can be formed integratedly with the wire 14 by well-known methods such as dispensing or screen printing.

To one end of the wire 15, a counter electrode 17 is connected. The counter electrode 17 is provided so as to surround the electrode chip 12 and the reference electrode 16. The counter electrode 17 can be, for example, an electrode formed of a metal such as Pt, Au, Cu, Pd, Ni, or Ag, or a carbon electrode. The counter electrode 17 is formed integratedly with the wire 15 by, for example, a subtractive method or a semi-additive method. The counter electrode 17 may be formed separately from the wire 15 and be connected to the wire 15 through a conductive bonding member such as a conductive paste.

The wires 13 to 15 are covered with a waterproof member 20 formed of, for example, insulative resin so that when urine is supplied to the sensor 10, the urine does not come into contact with the wires 13 to 15.

As illustrated in FIG. 2, the electrode chip 12 includes a diamond thin film (diamond film) 121 and a base material 122. The electrode chip 12 is used in a state in which urine is in contact with the diamond film 121. The electrode chip 12 including the diamond film 121 may also be referred to as a diamond electrode.

The diamond film 121 causes an electrochemical reaction of uric acid in the urine at an exposed surface (surface) 121a thereof. That is, in the sensor 10 including an electrode group including the electrode chip 12 (working electrode), the reference electrode 16, and the counter electrode 17, when urine has been supplied to the sensor 10, if a predetermined voltage is applied to the electrode group in a state in which the urine is in contact with the electrode group, a redox reaction of uric acid occurs at the exposed surface 121a of the diamond film 121.

The diamond film 121 is a polycrystalline film formed of diamond or diamond-like carbon (DLC). The diamond film 121 is provided on at least one of the surfaces of the base material 122 described later. In this description, the surface of the base material 122 on which the diamond film 121 is provided may also be referred to as a top surface of the base material 122. The diamond film 121 is provided over the entirety of the top surface of the base material 122. The diamond film 121 can be grown (deposited or synthesized) using, for example, a Chemical Vapor Deposition (CVD) method, or a Physical Vapor Deposition (PVD) method. Exemplary CVD methods include a thermal filament (hot filament) CVD method using tungsten filaments and a plasma CVD method. Exemplary PVD methods include an ion beam method and an ionized evaporation method. The diamond film 121 can have a thickness of, for example, 0.5 µm or more and 10 µm or less, preferably 2 µm or more and 4 µm or less.

The diamond film 121 can contain an element (dopant) such as boron (B) at a concentration of, for example, 1 × 10¹⁹ cm⁻³ or more and 1 × 10²² cm⁻³ or less, preferably 1 × 10²⁰ cm⁻³ or more and 5 × 10²¹ cm⁻³ or less. That is, the diamond film 121 can be p-type. B-concentration in the diamond film 121 can be measured, for example, by secondary ion mass spectrometry (SIMS).

The base material 122 is formed of a conductive material other than diamond or DLC. In other words, the base material 122 comprises a conductive material (dissimilar material) different from the material of the diamond film 121. For example, the base material 122 can be a base material formed using silicon (Si) alone or a Si compound, i.e. can be a conductive base material containing Si. Specifically, a Si substrate can be used as the base material 122. As the Si substrate, a single-crystal Si substrate, a polycrystalline Si substrate, a silicon carbide substrate (SiC substrate) can be used, for example.

The base material 122 can have a thickness of, for example, 350 µm or more. Accordingly, commercially available Si substrates can be used, such as single-crystal Si substrates or polycrystalline Si substrates with a diameter of 6 inches or 8 inches as the base material 122 directly without having to adjust the thickness by back lapping. As a result, the productivity of the electrode chip 12 can be increased and manufacturing cost can be reduced. Although the upper limit of the thickness of the base material 122 is not particularly limited, the thickness of Si substrates currently and generally available on the market is about 775 µm for a single crystal Si substrate with a diameter of 12 inches. Accordingly, the upper limit of the thickness of the base material 122 for the current state of the art can be, for example, about 775 µm.

Similarly to the diamond film 121, the base material 122 can contain an element such as B at a predetermined concentration and be a p-type. The B concentration in the base material 122 can be, for example, 5 × 10¹⁸ cm⁻³ or more and 1.5 × 10²⁰ cm⁻³ or less, preferably 5 × 10¹⁸ cm⁻³ or more and 1.2 × 10²⁰ cm⁻³ or less. When the B concentration in the base material 122 is within the aforementioned range, the specific resistance of the base material 122 can be reduced while also avoiding a decrease in the production yield and degradation in the performance of the base material 122.

The outline of the electrode chip 12 is rectangular, for example, square in a planar view. The electrode chip 12 can have a plane area of, for example, 1 mm² or more and 25 mm² or less. The plane area of the electrode chip 12 is the area of the electrode chip 12 when viewed from vertically above the surface thereof on which the diamond film 121 is provided. When the plane area of the electrode chip 12 is 1 mm² or more, the electrode chip 12 can easily be produced accurately and stably by a method involving breakage, described later. Moreover, deterioration in the handling property of the electrode chip 12 and deterioration in the mounting stability of the electrode chip 12 can also be suppressed. When the plane area of the electrode chip 12 is 25 mm² or less, an increase in the size of the sensor 10 can be avoided, that is, a smaller sensor 10 can be obtained.

The electrode chip 12 is secured onto the substrate 11 through a conductive bonding member 18 in such a manner that the diamond film 121 is on the upper side (i.e. the base material 122 opposes the substrate 11). The electrode chip 12 is electrically connected to the wire 13 through the conductive bonding member 18. As the conductive bonding member 18, a conductive paste (conductive adhesive) or a conductive tape can be used, for example.

An insulating resin 19 is provided so as to expose at least part of the diamond film 121. The insulating resin 19 is provided, for example, by applying an insulating resin (e.g. epoxy-based insulating resin) in liquid form to cover the entirety of side surfaces of the base material 122 and the conductive bonding member 18, and then curing the insulating resin in liquid form by heating or ultraviolet irradiation. In this way, the insulating resin 19 seals (at least) part of a surface of a conductive member included in the electrode chip 12. Specifically, the conductive bonding member 18 and the surface of the conductive member (i.e. the side surfaces of the base material 122) of the electrode chip 12 other than the diamond film 121 are resin sealed by the insulating resin 19.

On the exposed surface 121a of the diamond film 121 (the surface of diamond film 121), when the insulating resin 19 is provided, more specifically when the insulating resin in liquid form is cured by heating or ultraviolet irradiation, organic matters with various molecular weights volatilized from the resin adhere. As described above, when organic matter with a molecular weight of 100 or more (also referred to as "polymer organic matter" below) adheres to the exposed surface 121a by a certain amount or more, the protein in the urine reacts with the polymer organic matter when the urine comes into contact with the exposed surface 121a, and a membrane (protein membrane) is formed (rapidly) on the surface of the diamond film 121. This membrane becomes an obstruction in the uric acid reaching the exposed surface 121a, resulting in a decrease in the uric acid concentration measurement accuracy.

Thus, in this embodiment, after the insulating resin 19 is cured by heating or ultraviolet irradiation, polymer organic matter is removed from the exposed surface 121a so as to clean the exposed surface 121a. In other words, the adherence amount of polymer organic matter on the exposed surface 121a is made to be a certain amount or less, and the exposed surface 121a is made into a clean surface. Specifically, when the exposed surface 121a is measured by time-of-flight secondary ion mass spectrometry (TOF-SIMS), an integrated value B_{L} per unit area of ionic strength of the organic matter with a molecular weight of 100 or more (this integrated value will also be referred to as "integrated value B_{L}" below) is 1.2 × 10⁶ cts/sec or less, preferably 6.3 × 10⁵ cts/sec or less, more preferably 4.5 × 10⁵ cts/sec. The polymer organic matter adhering to the exposed surface 121a is volatile matter from the insulating resin. Therefore, in the TOF-SIMS measurement, peaks of organic ions such as C₇H₇O and Si₂C₅H₁₅O as polymer organic matter are detected.

Note that in TOF-SIMS, fragments are generated when primary ions collide with polymer organic matter. Due to the generation of fragments, the ionic strength (signal intensity) obtained by TOF-SIMS is smaller by the amount of these fragments than the ionic strength expected to be obtained due to the polymer organic matter adhering to the exposed surface 121a. In other words, the ionic strength attributable to the polymer organic matter obtained by TOF-SIMS is usually an ionic strength that is less than the concentration of the polymer organic matter adhering to the exposed surface 121a. Thus, it is difficult to quantitatively ascertain the molecular weight of the polymer organic matter actually adhering to the exposed surface 121a. However, the present inventors have found that even when fragments are generated during TOF-SIMS measurement, by adjusting the manufacturing process so that the ionic strength of the polymer organic matter is measured under a predetermined condition, the integrated value B_{L} per unit area of the ionic strength is calculated, and the value thereof is made to be at or below a predetermined threshold, the risk of the aforementioned membrane being formed can be avoided. This knowledge was found by the present inventors for the first time.

TOF-SIMS measurement can be performed using, for example, TOF-SIMS M6 manufactured by IONTOF. The following conditions are exemplary measurement conditions for TOF-SIMS.

### <Measurement Conditions>

Acceleration voltage: 30 kVDOSE: 1.3E + 10 (ions/cm²)
Beam diameter: 3 to 4 µm
Measurement area: 500 µm square
Total number of scans: 16 scans
Primary ion source: Bi³⁺⁺

As described above, when the integrated value B_{L} is 1.2 × 10⁶ cts/sec or less, formation of the aforementioned membrane on the exposed surface 121a can be suppressed even when the test liquid contains protein, as in the case of urine. Moreover, even when the aforementioned membrane is formed on the exposed surface 121a, a case in which the entirety of the exposed surface 121a is covered with the aforementioned membrane can be avoided. As a result, uric acid can reliably reach the exposed surface 121a and therefore accurate measurement of uric acid concentration can be performed. That is, a decrease in the uric acid concentration measurement accuracy can be avoided. On the other hand, when the integrated value B_{L} exceeds 1.2 × 10⁶ cts/sec, the aforementioned membrane is formed on the exposed surface 121a and the detection target cannot reach the exposed surface 121a. Therefore, when the uric acid concentration in the urine is measured by cyclic voltammetry (CV), the resulting cyclic voltammogram does not show a peak (peak current cannot be confirmed) and therefore there may be cases where the uric acid concentration cannot be measured.

Preferably, the integrated value B_{L} is 6.3 × 10⁵ cts/sec or less; if so, the formation of the aforementioned membrane can be reliably suppressed and a decrease in the uric acid concentration measurement accuracy can be reliably avoided. More preferably, the integrated value B_{L} is 4.5 × 10⁵ cts/sec or less; if so, the formation of the aforementioned membrane can be even more reliably suppressed and a decrease in the uric acid concentration measurement accuracy can be even more reliably avoided.

With a decrease in the integrated value B_{L}, i.e. with a smaller amount of polymer organic matter adhering to the exposed surface 121a, the formation of the aforementioned membrane can be suppressed more and the uric acid concentration can be measured with higher accuracy. Thus, the lower limit of the integrated value B_{L} is not particularly limited. However, even with thorough cleaning of the exposed surface 121a, in reality it is extremely difficult to reduce the integrated value B_{L} down to 0 (zero). The present inventors have confirmed that with the current technology, the integrated value B_{L} can be reduced to, for example, 3.5 × 10⁵ cts/sec.

As described above, in order to accurately measure the uric acid concentration, it is necessary to reduce the adherence amount of polymer organic matter on the exposed surface 121a. However, diamond is a hydrophobic material and therefore has poor liquid contact properties (hydrophilicity) and urine does not easily adhere to the exposed surface 121a with stability. Thus, even when the adherence amount of polymer organic matter on the exposed surface 121a is reduced, the uric acid concentration measurement accuracy may be low. On the other hand, there are a lot of organic matters with a molecular weight of less than 100 (also referred to as "low-molecular-weight organic matters" below) that are hydrophilic. Moreover, unlike polymer organic matters, low-molecular-weight organic matters do not cause unfavorable effects such as formation of the aforementioned membrane through reaction with protein in urine. Thus, it is preferable to have a certain amount of (some) low-molecular-weight organic matter adhering to the exposed surface 121a from the viewpoint of enhancing liquid contact properties with respect to urine. For example, it is preferable that when the exposed surface 121a is measured by TOF-SIMS, an integrated value Bs per unit area of ionic strength of organic matter with a molecular weight of less than 100 (this integrated value will also be referred to as "integrated value Bs" below) be 1.9 × 10⁶ cts/sec or more. In the TOF-SIMS measurement, peaks of organic ions such as C₇H₇, C₂H₅, C₂H₃O, C₃H₃O, and C₄H₅O as low-molecular-weight organic matter are detected.

As described above, in TOF-SIMS measurement, a certain amount of fragments are generated when primary ions collide with polymer organic matter. When the molecular weight of the fragments is less than 100, the ionic strength (signal intensity) obtained by TOF-SIMS when measuring the adherence amount of low-molecular-weight organic matter is the total strength of the ionic strength attributable to the fragments plus the ionic strength attributable to low-molecular-weight organic matter. In other words, the ionic strength attributable to the low-molecular-weight organic matter obtained by TOF-SIMS is usually an ionic strength that is equal to or more than the concentration of the low-molecular-weight organic matter adhering to the exposed surface 121a. Thus, it is difficult to quantitatively ascertain the adherence amount of the low-molecular-weight organic matter that has actually adhered. However, the present inventors have found that even when fragments are generated during TOF-SIMS measurement, by adjusting the manufacturing process so that the ionic strength of the low-molecular-weight organic matter is measured under a predetermined condition, the integrated value Bs per unit area of the ionic strength is calculated, and the value thereof is made to be a predetermined threshold or more, a diamond film 121 with high liquid contact properties can be obtained. This knowledge was found by the present inventors for the first time. The measurement conditions for TOF-SIMS are similar to the measurement conditions for the integrated value B_{L} described above.

When the integrated value Bs is, for example, 1.9 × 10⁶ cts/sec or more, the liquid contact properties (liquid contact stability) of (the exposed surface 121a of) the diamond film 121 can be enhanced, and urine can be stably adhered to the exposed surface 121a. As a result, the uric acid concentration can be measured even more accurately. On the other hand, when the integrated value Bs is, for example, less than 1.9 × 10⁶ cts/sec, there are cases where the effect of enhancing the liquid contact properties of the exposed surface 121a cannot be obtained. Therefore, even when the adherence amount of polymer organic matter on the exposed surface 121a is reduced, the uric acid concentration measurement accuracy may be low.

Preferably, the integrated value Bs is 2.4 × 10⁶ cts/sec or more; if so, the aforementioned liquid contact properties can be reliably enhanced. More preferably, the integrated value Bs is 3.2 × 10⁶ cts/sec or more; if so, the aforementioned liquid contact properties can be even more reliably enhanced.

Theoretically, the higher the integrated value Bs, the higher the aforementioned liquid contact properties. However, as described above, the ionic strength obtained by TOF-SIMS when measuring the adherence amount of low-molecular-weight organic matter is the total strength of the ionic strength attributable to the fragments plus the ionic strength attributable to low-molecular-weight organic matter. Therefore, a higher integrated value Bs does not necessarily result in an increase in the amount of low-molecular-weight organic matter adhering to the exposed surface 121a. For example, in some cases, due to a large adherence amount of polymer organic matter, the ionic strength attributable to the fragments increases, and as a result, the integrated value Bs increases. In this way, when the integrated value Bs is too high, the integrated value B_{L} may not be 1.2 × 10⁶ cts/sec or less. In order to reliably obtain a sensor 10 for which the integrated value B_{L} at the exposed surface 121a is 1.2 × 10⁶ cts/sec or less, the upper limit of the integrated value Bs can be set to, for example, 4.8 × 10⁶ cts/sec.

Preferably, the ratio (B_{L}/B_{S}) of the integrated value B_{L} to the integrated value Bs is, for example, 0.3 or less. If so, a sensor 10 for which the integrated value B_{L} at the exposed surface 121a is 1.2 × 10⁶ cts/sec or less can be obtained. On the other hand, when the value of B_{L}/B_{S} is more than 0.3, the integrated value B_{L} at the exposed surface 121a may become more than 1.2 × 10⁶ cts/sec in some cases.

More preferably, the value of B_{L}/B_{S} is 0.23 or less. If so, a sensor 10 for which, for example, the integrated value B_{L} at the exposed surface 121a is 1.2 × 10⁶ cts/sec or less and the integrated value Bs at the exposed surface 121a is 1.9 × 10⁶ cts/sec or more can be reliably obtained.

Preferably, the lower limit of B_{L}/B_{S} is as close to 0 (zero) as possible. However, as described above, with the current technology, it is extremely difficult to actually reduce the integrated value B_{L} down to 0 (zero). The present inventors have confirmed that with the current technology, the value of B_{L}/B_{S} can be reduced to 0.11.

By cleaning the exposed surface 121a, the exposed surface 121a is oxygen-terminated. In this way, a change in the sensitivity of the sensor 10 over time can be suppressed and the sensor sensitivity can be stabilized over a long period of time. As a result, a sensor 10 that remains reliable over a long period of time can be obtained. The oxygen termination of the exposed surface 121a (the surface of the diamond film 121) can be evaluated, for example, by X-ray photoelectron spectroscopy (XPS).

By cleaning the exposed surface 121a, an oxide layer 19a is formed on the surface of the insulating resin 19. This "oxide layer 19a" is a layer formed by oxidation of the surface (surface layer) of the insulating resin 19 and is, for example, a layer containing more C=O bonds and O-H bonds than the surface of resin before curing and the deep layer (parts other than the surface layer) of the insulating resin 19 after curing. The oxide layer 19a can be evaluated, for example, by Fourier transform infrared spectroscopy (FTIR).

The oxide layer 19a functions as a block layer that, when the organic matter (organic matter including polymer organic matter) is removed from the insulating resin 19 that has not been oxidized, prevents the removed organic matter from reaching the exposed surface 121a. Thus, due to the formation of the oxide layer 19a, the exposed surface 121a can be kept clean for a long period of time. For example, even when the sensor 10 is stored in a sealed package for a long period of time in a condition in which temperature may easily rise, the organic matter having been removed from the insulating resin 19 can be prevented from adhering to the exposed surface 121a during storage, for example.

During the storage of the sensor 10, for example, the organic matter removed from the oxide layer 19a may adhere to the exposed surface 121a. In general, the molecular weight of resin is reduced by oxidation, and therefore the molecular weight of the oxide layer 19a is lower than the molecular weight of the insulating resin 19 before oxidation. Thus, even when the organic matter removed from the oxide layer 19a adheres to the exposed surface 121a, the organic matter almost never causes unfavorable effects such as forming the aforementioned membrane by reacting with protein in urine, unlike the organic matter removed from the insulating resin 19 that has not been oxidized.

It is known that the strength of the insulating resin 19 decreases when the insulating resin 19 is oxidized. In this embodiment, the oxide layer 19a is formed by oxidizing at least the surface (surface layer) alone of the insulating resin 19, and therefore a decrease in the strength of the insulating resin 19 can be suppressed to such an extent that the use of the sensor 10 is not affected.

The oxide layer 19a can have a thickness of, for example, 1 nm or more, preferably 2 nm or more. When the thickness of the oxide layer 19a is 1 nm or more, the entire surface of the insulating resin 19 can be covered with the oxide layer 19a. When the thickness of the oxide layer 19a is 2 nm or more, the entire surface of the insulating resin 19 can be covered with the oxide layer 19a reliably. As a result, the exposed surface 121a can be reliably kept clean for a long period of time. When the oxide layer 19a is excessively thick, the strength of the insulating resin 19 decreases and the use of the sensor 10 may be affected in some cases. On condition that the oxide layer 19a can cover the entire surface of the insulating resin 19, it is preferable to minimize the thickness of the oxide layer 19a.

### <2> Method for Manufacturing Electrochemical Sensor

Next, a method for manufacturing the sensor 10 described above will be described.

In a method for manufacturing the sensor 10 in this embodiment, there are performed
growing the diamond film 121 on the base material 122 (Step A),
processing a stack of the diamond film 121 and the base material 122 into a chip shape and obtaining the electrode chip 12 including the diamond film 121 on a surface of the electrode chip 12 (Step B),
using the conductive bonding member 18 to secure the electrode chip 12 onto the substrate 11 (Step C),
applying an insulating resin in liquid form in such a manner that at least part of the diamond film 121 is exposed, and then curing the insulating resin in liquid form by heating or ultraviolet irradiation to provide the insulating resin 19 (Step D),
and performing oxidization processing on the exposed surface 121a to oxidize polymer organic matter having volatilized from the insulating resin 19 and adhered to the exposed surface 121a due to Step D, and removing the polymer organic matter from the exposed surface 121a (Step E).

### <Step A>

As the base material 122, a conductive substrate, such as a Si substrate having a circular outline in a planar view, is prepared. Then, among the two main surfaces of the base material 122, the surface on which the diamond crystal will be grown (deposited) (also referred to as "crystal-growing surface" below) is subjected to seeding processing or scratching processing. The seeding processing refers to processing of attaching diamond grains (seeds) onto the crystal-growing surface by applying a solution (dispersion liquid) on the crystal-growing surface, or by immersing the base material 122 in the dispersion liquid. Here, the dispersion liquid is a liquid in which the diamond grains (preferably diamond nanoparticles) of, for example, about several nanometers to several tens of micrometers are dispersed. The scratching processing refers to processing of making scratches on the crystal-growing surface using, for example, diamond abrasive grains (diamond powder) of about several micrometers.

After the seeding processing or the scratching processing is completed, the diamond crystal is grown on the crystal-growing surface of the base material 122 by, for example, a thermal filament CVD method using tungsten filaments, thereby forming the diamond film 121.

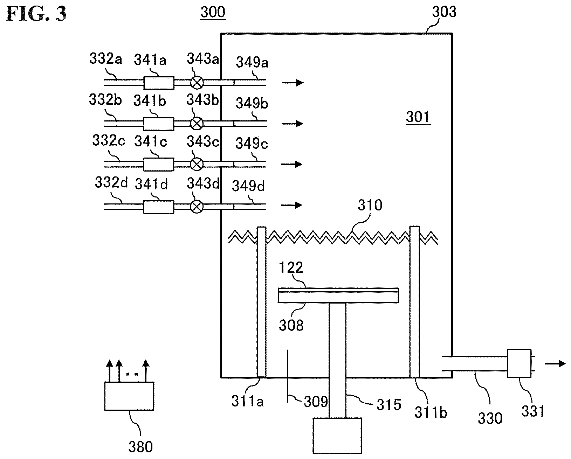

The diamond film 121 can be grown using, for example, a thermal filament CVD apparatus 300 illustrated in FIG. 3. The thermal filament CVD apparatus 300 includes an airtight container 303 which comprises a heat-resistant material such as quartz and in which a growth chamber 301 is constructed. A susceptor 308 that holds the base material 122 is provided in the growth chamber 301. To a sidewall of the airtight container 303, there are connected a gas supply pipe 332a that supplies nitrogen (N₂) gas into the growth chamber 301, a gas supply pipe 332b that supplies hydrogen (H₂) gas, a gas supply pipe 332c that supplies methane (CH₄) gas or ethane (C₂H₆) gas as a carbon (C)-containing gas, and a gas supply pipe 332d that supplies trimethylboron (B(CH₃)₃, abbreviated as TMB) gas, trimethyl borate (B(OCH₃)₃) gas, triethyl borate (B(C₂H₅O)₃) gas, or diborane (B₂H₆) gas as a boron (B)-containing gas. To the gas supply pipes 332a to 332d, there are respectively provided flowrate controllers 341a to 341d and valves 343a to 343d in order from the upstream side of the gas flow. To downstream ends of the gas supply pipes 332a to 332d, there are respectively connected nozzles 349a to 349d that supplies gas supplied through the gas supply pipes 332a to 332d into the growth chamber 301. To another sidewall of the airtight container 303, there is provided an exhaust pipe 330 that exhausts the growth chamber 301. To the exhaust pipe 330, there is provided a pump 331. In the airtight container 303, there is provided a temperature sensor 309 that measures a temperature in the growth chamber 301. In the airtight container 303, there are provided a tungsten filament 310, and a pair of electrodes (e.g. molybdenum (Mo) electrodes) 311a, 311b that heats the tungsten filament 310. Each member included in the thermal filament CVD apparatus 300 is connected to a controller 380 configured as a computer, and is configured so that processing procedures and processing conditions described later are controlled with a program executed on the controller 380.

The diamond film 121 can be grown using the thermal filament CVD apparatus 300 described above according to the following processing procedures, for example. First, the base material 122 is loaded (installed) in the airtight container 303, and held on the susceptor 308. Then, H₂-gas is supplied into the growth chamber 301 while exhausting the growth chamber 301. A current is passed between the electrodes 311a and 311b to start heating of the tungsten filament 310. As the tungsten filament 310 is heated, the base material 122 held on the susceptor 308 is also heated. After the tungsten filament 310 reaches a desired temperature, a pressure in the growth chamber 301 reaches a desired pressure, and an atmosphere in the growth chamber 301 becomes a desired atmosphere, then a C-containing gas (e.g. CH₄-gas) and a B-containing gas (e.g. TMB-gas) are supplied into the growth chamber 301. As CH₄-gas and TMB-gas supplied into the growth chamber 301 pass through the tungsten filament 310 heated to an elevated temperature, they are decomposed (thermally decomposed) to generate active species such as methyl radicals (CH₃*). As these active species and the like are supplied on the base material 122, the diamond crystal grows.

The following conditions are exemplified as the conditions for growing the diamond film 121. The diamond film 121 growth time may be adjusted according to the diamond film 121 thickness.
Base material temperature: 600 °C or more and 1000 °C or less, preferably, 650 °C or more and 800 °C or less
Filament temperature: 1800 °C or more and 2500 °C or less, preferably, 2000 °C or more and 2200 °C or less
Pressure in growth chamber: 5 Torr or more and 50 Torr or less (665 Pa or more and 6650 Pa or less), preferably 10 Torr or more and 35 Torr or less (1330 Pa or more and 4655 Pa or less)
Ratio between partial pressure of CH₄-gas and partial pressure of TMB-gas (TMB/CH₄): 0.003 % or more and 0.8 % or less
Ratio between H₂-gas and CH₄-gas (CH₄/H₂): 2 % or more and 5 % or less
Growth time: 30 minutes or more and 120 minutes or less, preferably 60 minutes or more and 100 minutes or less

By growing the diamond crystal under the conditions described above, the diamond crystal grows with the diamond grains adhering to the crystal-growing surface of the base material 122 and the scratches on the crystal-growing surface serving as nuclei, and a p-type diamond film 121 with a B concentration of, for example, 1 × 10¹⁹ cm⁻³ or more and 1 × 10²² cm⁻³ or less can be grown on the crystal-growing surface of the base material 122.

### <Step B>

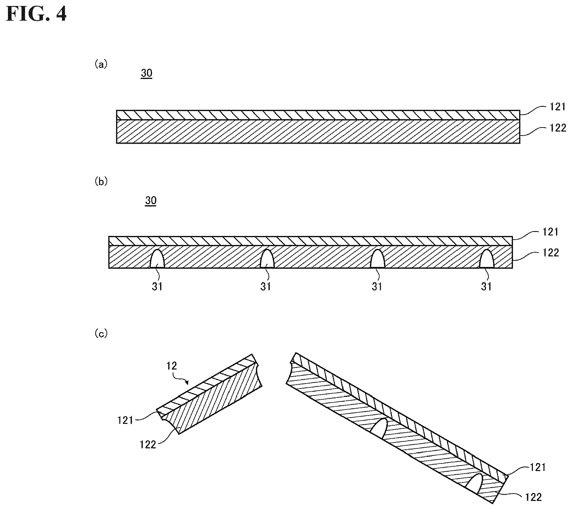

After Step A is completed, Step B is performed. That is, a stack (stacked wafer 30) of the diamond film 121 and the base material 122 (as illustrated in FIG. 4(a)) is formed into a chip shape and the electrode chip 12 including the diamond film 121 on a surface of the electrode chip 12 is obtained.

Specifically, concave grooves 31 (e.g. scribed grooves) are formed starting from a rear surface (base material 122 side) of the stacked wafer 30, as illustrated in FIG. 4(b). The grooves 31 can be formed using a known method such as laser machining (e.g. laser scribing or laser dicing), mechanical machining, or etching, for example. The grooves 31 are formed such that the grooves 31 do not penetrate through the base material 122 in the thickness direction, i.e. do not reach the diamond film 121. For example, the grooves 31 are formed such that the thickness of the thinnest part of the base material 122 is, for example, 10 µm or more and 80 µm or less. In this way, degradation of the diamond film 121 can be suppressed, and as a result, a decrease in the accuracy of measuring the concentration of uric acid (detection target) can be more reliably avoided in the sensor 10 using the electrode chip 12. This "degradation of the diamond film 121" means, for example, that the sp³ bond of the diamond film 121 changes to an sp² bond, i.e. graphitization of the diamond.

Next, as illustrated in FIG. 4(c), the stacked wafer 30 is folded along the groove 31 in a direction in which the diamond film 121 is folded outward to break the base material 122. In this way, the electrode chip 12 including the diamond film 121 and the base material 122 can be obtained.

In Step B, it is also conceivable to form the grooves 31 starting from a front surface (the diamond film 121 side) of the stacked wafer 30. However, since the diamond film 121 is very hard, it is difficult to form the grooves 31 from the diamond film 121 side by, for example, laser machining or mechanical machining.

In Step B, it is also conceivable to obtain the electrode chip 12 by forming the stacked wafer 30 into a predetermined shape by, for example, dry etching, instead of using the breakage-based method described above. However, it is very difficult to form the stacked wafer 30 having the hard diamond film 121 into a predetermined shape by dry etching, etc. In addition, dry etching may generate degraded zones in the diamond film 121.

### <Step C>

After Step B is completed, Step C is performed. That is, using the conductive bonding member 18, the electrode chip 12 is secured onto the substrate 11.

Specifically, first, the substrate 11 is prepared. Then, on the substrate 11, there are provided: the wires 13 to 15 each having a predetermined pattern; the reference electrode 16 electrically connected to the wire 14; and the counter electrode 17 electrically connected to the wire 15. (Note that a substrate 11 may also be prepared on which the wires 13 to 15, the reference electrode 16, the counter electrode 17, etc. are already provided in advance.) Then, the conductive bonding member 18 is provided on the substrate 11 so as to electrically connect to the wire 13, and the electrode chip 12 is placed on the conductive bonding member 18. At this time, the electrode chip 12 is placed in such a manner that the diamond film 121 is on the upper side (i.e. the base material 122 opposes the substrate 11). In this way, the electrode chip 12 is secured onto the substrate 11 and electrically connected to the wire 13 through the conductive bonding member 18.

### <Step D>

After Step C is completed, Step D is performed. That is, the insulating resin in liquid form is applied around the electrode chip 12 secured onto the substrate 11 such that at least part of the diamond film 121 is exposed, and then the insulating resin in liquid form is cured by heating or ultraviolet irradiation. As a result, the electrode chip 12 is brought into a state in which at least part of the diamond film 121 is exposed, and the entirety of the side surfaces of the base material 122 and the conductive bonding member 18 are resin sealed with the insulating resin 19.

By performing Step D, organic matters with various molecular weights that have been volatilized (removed) from the insulating resin 19 will adhere to the exposed surface 121a.

In the explanation described above, the electrode chip 12 is secured by the conductive bonding member 18 and the resin sealing is provided using the insulating resin 19. However, the electrode chip 12 may preliminarily be tacked onto the substrate 11 using the conductive bonding member 18, and then the insulating resin 19 may be used to both secure the electrode chip 12 and provide the resin sealing. In other words, the insulating resin 19 may have both the function of securing the electrode chip 12 and the function of providing the resin sealing. In this case, the securing of the electrode chip 12 and the resin sealing are both performed in Step D.

### <Step E>

After Step D is completed, Step E is performed. That is, the exposed surface 121a is cleaned. Specifically, oxidization processing is performed on the exposed surface 121a of the diamond film 121 to oxidize the polymer organic matter in the organic matter having volatilized from the insulating resin 19 (insulating resin in liquid form) and adhered to the exposed surface 121a due to Step D, thereby removing the polymer organic matter from the exposed surface 121a. As the oxidation processing, it is preferable to use gas-phase etching. Examples of the gas-phase etching include corona treatment, atmospheric plasma treatment (Oz plasma treatment), and UV ozone treatment.

The following conditions are exemplified as conditions for the oxidation processing (e.g. atmospheric plasma treatment):
Processing time: 30 seconds or more and 15 minutes or less
Applied voltage: 10 kV
Gap length: 3 mm

By performing the oxidization processing described above, the polymer organic matter having adhered to the exposed surface 121a can be oxidized and the polymer organic matter can be removed from the exposed surface 121a. That is, by performing the oxidization processing described above, the integrated value B_{L} described above at the exposed surface 121a can be reduced to 1.2 × 10⁶ cts/sec or less.

In addition, by performing the oxidization processing described above, removal of the low-molecular-weight organic matter from the exposed surface 121a can be suppressed and a certain amount of low-molecular-weight organic matter can be kept adhered to the exposed surface 121a. That is, the integrated value Bs described above at the exposed surface 121a can be kept at, for example, 1.9 × 10⁶ cts/sec or more.

By performing the oxidization processing described above, the ratio (B_{L}/B_{S}) of the integrated value B_{L} to the integrated value Bs can be made to be, for example, 0.3 or less.

In addition, by performing the oxidization processing described above, the exposed surface 121a is oxygen-terminated. Further, the surface (surface layer) of the insulating resin 19 is oxidized and the oxide layer 19a is formed.

In Step E, it is also conceivable to perform wet etching on the exposed surface 121a to remove the polymer organic matter having adhered to the exposed surface 121a. However, in this case, not only the polymer organic matter but also the low-molecular-weight organic matter will be removed. Thus, there are cases where the integrated value Bs cannot be kept at, for example, 1.9 × 10⁶ cts/sec or more, or the aforementioned B_{L}/B_{S} value cannot be made to be 0.3 or more. As a result, the liquid contact properties of the diamond film 121 may be low, and the uric acid concentration measurement accuracy may decrease.

In Step E, it is also conceivable to clean the exposed surface 121a using, for example, a surfactant to remove the polymer organic matter having adhered to the exposed surface 121a. However, the present inventors have confirmed that in this case, the polymer organic matter cannot be removed sufficiently.

According to the above, a sensor 10 for which the integrated value B_{L} described above at the exposed surface 121a is 1.2 × 10⁶ cts/sec or less can be obtained.

### <3> Effects

According to the embodiments above, one or more of the following effects can be obtained.
(a) The integrated value B_{L} of the polymer organic matter (organic matter with a molecular weight of 100 or more) when the exposed surface 121a is measured by TOF-SIMS is 1.2 × 10⁶ cts/sec or less, and therefore reaction of protein in urine with the polymer organic matter can be suppressed and formation of a membrane of the protein on the exposed surface 121a can be suppressed. In other words, the risk of the aforementioned membrane being formed on the exposed surface 121a can be avoided even when the test liquid contains protein, as in the case of urine. Moreover, even when the aforementioned membrane is formed, a case in which the entirety of the exposed surface 121a is covered with the aforementioned membrane can be avoided. As a result, uric acid can reliably reach the exposed surface 121a and therefore accurate measurement of uric acid concentration can be performed. In other words, by reducing the integrated value B_{L} described above at the exposed surface 121a to 1.2 × 10⁶ cts/sec or less, a decrease in the accuracy of measuring the concentration of a detection target can be avoided even when the test liquid contains protein (i.e. regardless of the type of test liquid).
(b) The integrated value Bs of the low-molecular-weight organic matter (organic matter with a molecular weight of less than 100) when the exposed surface 121a is measured by TOF-SIMS is at 1.9 × 10⁶ cts/sec or more, and therefore the liquid contact properties of the exposed surface 121a can be enhanced. As a result, the uric acid concentration can be measured even more accurately.
(c) The ratio (B_{L}/B_{S}) of the integrated value B_{L} to the integrated value Bs is 0.3 or less, and therefore a sensor 10 for which the integrated value B_{L} at the exposed surface 121a is 1.2 × 10⁶ cts/sec or less can be obtained.
(d) The exposed surface 121a is oxygen-terminated, and therefore a change in the sensitivity of the sensor 10 over time can be suppressed and the sensor sensitivity can be stabilized over a long period of time. In other words, a sensor 10 that remains reliable over a long period of time can be obtained.
   For the sensor 10 in which the exposed surface 121a is oxygen-terminated and a sensor in which the exposed surface 121a is hydrogen-terminated, the present inventors performed CV measurement of the uric acid concentration in urine immediately after the electrode chip 12 was mounted on the substrate 11 and one month after the mounting to measure peak intensity (peak current value). They confirmed that when the exposed surface 121a is oxygen-terminated, the ratio of the peak intensity (P2) one month after the mounting to the peak intensity (P1) immediately after the mounting (= (P2/P1) × 100) was approximately 98 %, i.e. the sensor sensitivity showed substantially no decrease even after the lapse of one month after the mounting. In contrast, they confirmed that when the exposed surface 121a is hydrogen-terminated, the aforementioned ratio (= (P2/P1) × 100) was approximately 90 %. In this way, the present inventors confirmed that compared to when the exposed surface 121a is hydrogen-terminated, the peak intensity in CV measurement is less prone to change when the exposed surface 121a is oxygen-terminated, i.e. sensor sensitivity is less prone to change over time when the exposed surface 121a is oxygen-terminated.
(e) The surface of the insulating resin 19 is oxidized, that is, the oxide layer 19a is formed on the surface of the insulating resin 19, and therefore adherence of the organic matter removed from the unoxidized insulating resin 19 to the exposed surface 121a can be suppressed during storage, etc. As a result, the exposed surface 121a can be kept clean for a long period of time.

### <4> Modified example

The embodiments above can be modified as in the following modified examples. In the explanations of the following modified examples, components identical to those described above are marked with the same sign or numeral, and explanation thereof is omitted. The embodiments above and the following modified examples can be combined arbitrarily.

### <Modified example 1>

In the embodiments described above, an example was described where the entirety of the side surfaces of the base material 122 is covered with the insulating resin 19. However, the embodiments are not limited thereto.

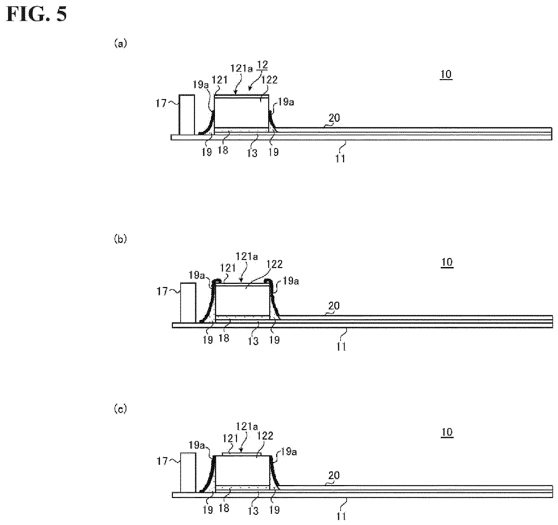

It is not necessary that the insulating resin 19 cover the entirety of the side surfaces of the base material 122; for example, the insulating resin 19 may be provided so as to cover at least part of the side surfaces of the base material 122 (e.g. the lower side of the base material 122 (the substrate 11 side)) and the conductive bonding member 18, as illustrated in FIG. 5(a). That is, the upper side of the side surfaces of the base material 122 (the diamond film 121 side) may be exposed. In this modified example as well, a similar effect to the embodiments described above can be obtained by having the integrated value B_{L} at the exposed surface 121a of 1.2 × 10⁶ cts/sec or less.

Even when part of the base material 122 is exposed, by performing Step E described above, the exposed surface of the base material 122 is oxidized, and as a result, the exposed surface of the base material 122 is deactivated. Thus, the occurrence of a redox reaction of the detection target on the exposed surface of the base material 122 can be avoided, and a decrease in the accuracy of measuring the detection target can be avoided. The present inventors have confirmed that at least in the range of the voltage applied to the sensor 10 (electrode group) during uric acid concentration measurement (e.g. a voltage range including a range of 0 V or more to 1 V or less), almost no redox reaction of uric acid occurs on the exposed surface of the side surfaces of the base material 122. However, from the perspective of reliably avoiding a decrease in the accuracy of measuring the concentration of the detection target, it is preferable that the insulating resin 19 be provided so as to cover the entirety of the side surfaces of the base material 122.

To present another example, as illustrated in FIG. 5(b), the insulating resin 19 may be provided so as to cover the entirety of the side surfaces of the base material 122 while also covering the side surfaces of the diamond film 121 and the periphery of the exposed surface 121a. In this way, (a) conductive member(s) in the electrode chip 12 other than the diamond film 121 can be more reliably resin sealed, and a decrease in the accuracy of measuring the concentration of the detection target can be suppressed even more reliably.

### <Modified example 2>

In the embodiments described above, an example was described where the diamond film 121 is provided on the entire top surface of the base material 122. However, the embodiments are not limited thereto. For example, as illustrated in FIG. 5(c), the diamond film 121 may be provided only on part of the top surface of the base material 122 and part of the top surface of the base material 122 may be exposed. In this modified example as well, a similar effect to the embodiments described above can be obtained by having the integrated value B_{L} at the exposed surface 121a of 1.2 × 10⁶ cts/sec or less.

In this modified example as well, similarly to Modified example 1, by performing Step E described above, the exposed surface of the base material 122 is oxidized, and as a result, the exposed surface of the base material 122 is deactivated. Thus, the occurrence of a redox reaction of the detection target on the exposed surface of the base material 122 can be avoided, and a decrease in the accuracy of measuring the detection target can be avoided.

However, from the perspective of reliably avoiding a decrease in the accuracy of measuring the concentration of the detection target, it is preferable that the surface of the base material 122 not be exposed. That is, in this modified example, it is preferable that the insulating resin 19 be provided so as to cover the entirety of the side surfaces of the base material 122 while also covering the portion of the top surface of the base material 122 where the diamond film 121 is not provided (this portion will also be referred to as the "exposed surface of the top surface of the base material 122" below). It is even more preferable that the insulating resin 19 be provided so as to cover the entirety of the side surfaces of the base material 122, the exposed surface of the top surface of the base material 122, the side surfaces of the diamond film 121, and the periphery of the exposed surface 121a of the diamond film 121.

### <Other Embodiments>

Specific embodiments of the present disclosure have been explained. However, the present disclosure is not limited to the embodiments described above, and can be variously modified within the scope of the gist of the disclosure.

For example, on the diamond film 121, there may be provided a functional membrane allowing only a predetermined detection target (e.g. uric acid) to pass through and/or a functional membrane reacting only with the detection target. Such functional membranes may be provided in the form of predetermined surface decoration membranes such as a membrane containing predetermined enzymes responding to an intended detection component, an ion exchange membrane, etc.

In the embodiments and modified examples described above, examples were explained where the test liquid is human urine and the detection target is uric acid, but this is not limiting. The test liquid may be liquids containing protein such as bodily fluid, blood, urine, etc. of an animal including human. This means that the sensor 10 (electrode chip 12) is used in such a manner that the test liquid containing bodily fluid, blood, or urine of an animal including human is in contact with the diamond film 121. Regarding the detection target, given that the voltage applied to the electrode group (at least between the electrode chip 12 and the counter electrode 17) is within a predetermined voltage range, the concentration of a variety of components (matters) other than uric acid can be measured by appropriately changing the voltage sweep conditions for cyclic voltammetry.

In the embodiments and modified examples described above, examples were explained where the electrode chip 12 is used as a working electrode of the sensor 10, but this is not limiting. The electrode chip 12 can also be used, for example, as an electrolytic electrode (e.g. electrolytic electrode for ozone generators) for electrolysis or synthesis of compounds or electrolysis of water, for example.

The sensor 10 may be, for example, a sensor that performs measurement by a two-electrode method. That is, the reference electrode 16 and the wire 14 may not be provided.

### EXAMPLES

Experimental results supporting the effects of the embodiments described above are described below.

### <Sample 1>

With Sample 1, a 6-inch Si substrate having a circular shape in a planar view was prepared as the base material, and among the main surfaces of the Si substrate, the surface on which a diamond crystal is to be grown (i.e. the crystal-growing surface) was subjected to predetermined scratching processing. Then, using the thermal filament CVD apparatus illustrated in FIG. 3 and tungsten filaments as the filaments, a diamond crystal was grown on the crystal-growing surface of the Si substrate having undergone the scratching processing to provide a diamond film having a predetermined B concentration, thereby producing a stacked wafer, i.e. a stack of the Si substrate and the diamond film.

Then, using a galvano optical laser, the rear surface (the surface on the opposite side from the surface on which the diamond film is provided) of the stacked wafer (Si substrate) was irradiated with a laser beam (532nm, 5W, 10kHz, spot diameter 2 µm) at a scanning speed of 10 cm/sec in a lattice form with a pitch of 2 mm, thereby forming lattice-shaped laser grooves (concave grooves). The number of scans with the laser beam was set to four times. Thereafter, breaking was performed along the laser grooves under the conditions of [break depression amount: 0.1 mm] and [break speed: 100 mm/s] to produce electrode chips with a size of 2 mm square from the stacked wafer.

On the substrate (plate-shaped member with insulating properties), two wires and a counter electrode connected to one of the two wires were provided. Then, the electrode chip was secured onto the substrate using a conductive paste in such a manner that the base material of the electrode chip faced the substrate and connected to the other one of the two wires. The two wires were then covered with insulative resin so that the wires were not exposed. Thereafter, the insulative resin in liquid form was applied in such a manner that at least part of the diamond film was exposed yet the base material of the electrode chip and the conductive paste were not exposed, then the insulative resin in liquid form was cured by heating to provide the insulating resin.

Next, oxidation processing (Oz plasma treatment) was performed on the exposed surface of the diamond film to clean the exposed surface. The sensor thus obtained was taken as Sample 1.

### <Samples 2 to 8>

For Samples 2 to 8, the oxidation processing time was changed from Sample 1. Conditions other than the oxidation processing time were identical to those of Sample 1.

### <Evaluation>

In Samples 1 to 8, the ionic strength of organic matter (polymer organic matter) with a molecular weight of 100 or more at the exposed surface of the diamond film was measured by TOF-SIMS under predetermined conditions, and the integrated value B_{L} thereof per unit area was calculated. Moreover, in Samples 1 to 8, the ionic strength of organic matter (low-molecular-weight organic matter) with a molecular weight of less than 100 at the exposed surface of the diamond film was measured by TOF-SIMS under predetermined conditions, and the integrated value Bs thereof per unit area was calculated. The measurement conditions for TOF-SIMS were the following.

### <Measurement Conditions for TOF-SIMS>

Acceleration voltage: 30 kVDOSE: 1.3E + 10 (ions/cm²)
Beam diameter: 3 to 4 µm
Measurement area: 500 µm square
Total number of scans: 16 scans
Primary ion source: Bi³⁺⁺

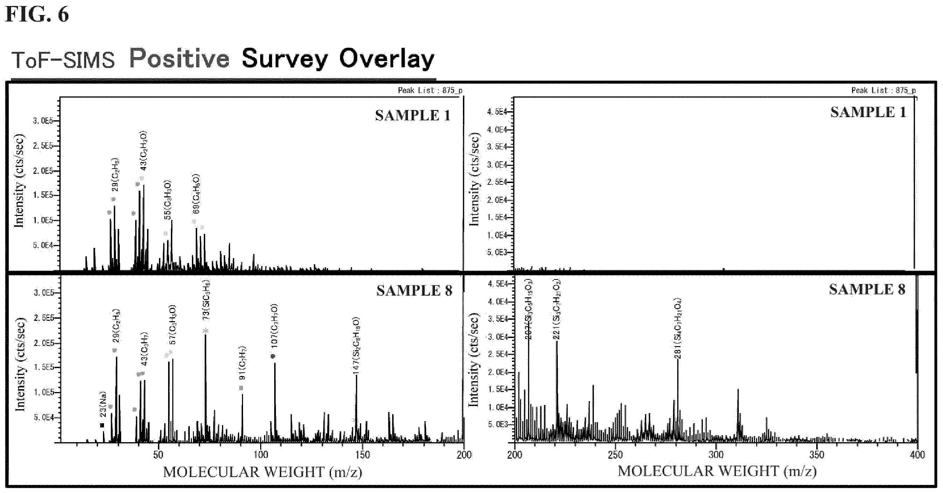

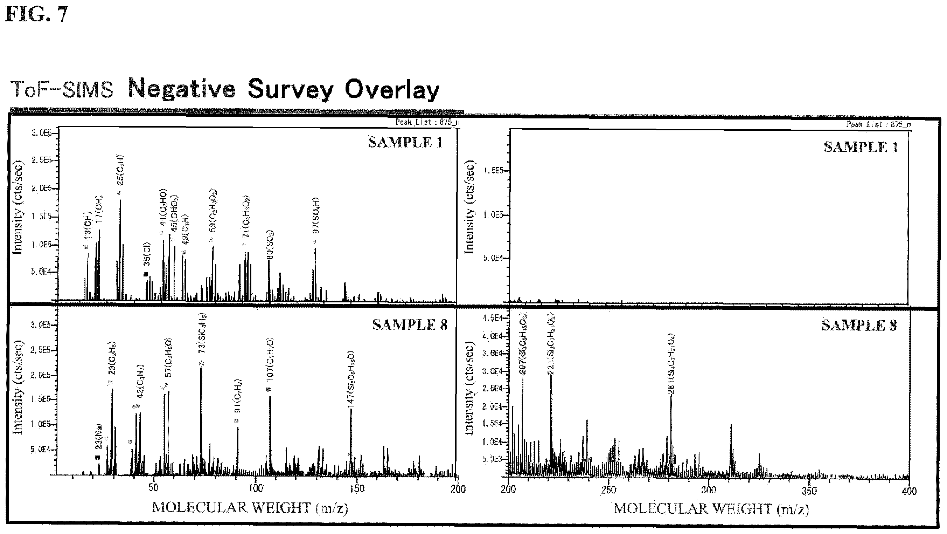

As examples, the results of TOF-SIMS evaluated for positive ions (cations) for Samples 1 and 8 are shown in FIG. 6, and the results of TOF-SIMS evaluated for negative ions for Samples 1 and 8 are shown in FIG. 7. The "results of TOF-SIMS evaluated for positive ions" mean the results of evaluating the ionic strength of organic ions of positive ions generated in the TOF-SIMS measurement. The "results of TOF-SIMS evaluated for negative ions" mean the results of evaluating the ionic strength of organic ions of negative ions generated in the TOF-SIMS measurement.

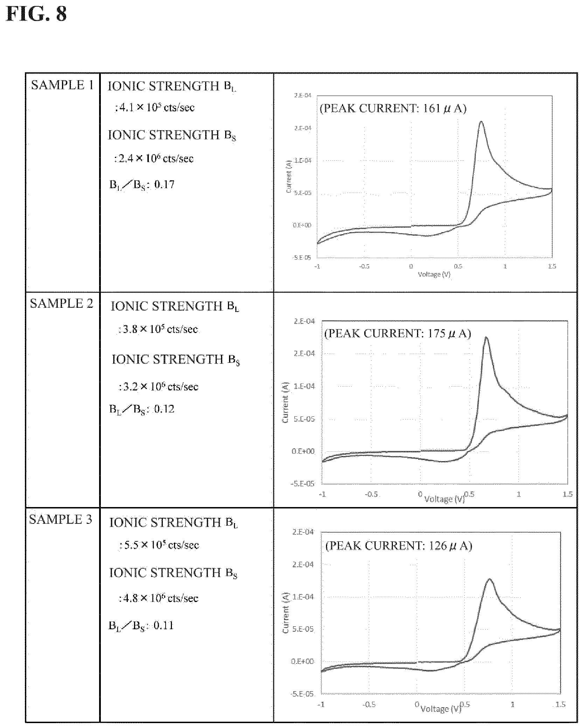

FIG. 8 to FIG. 10 show the results of measuring the integrated value B_{L} and the integrated value Bs for Samples 1 to 8. The ratio (B_{L}/B_{S}) of the integrated value B_{L} to the integrated value Bs was also calculated from the obtained integrated value B_{L} and integrated value Bs in Samples 1 to 8. These results are shown in FIG. 8 to FIG. 10.

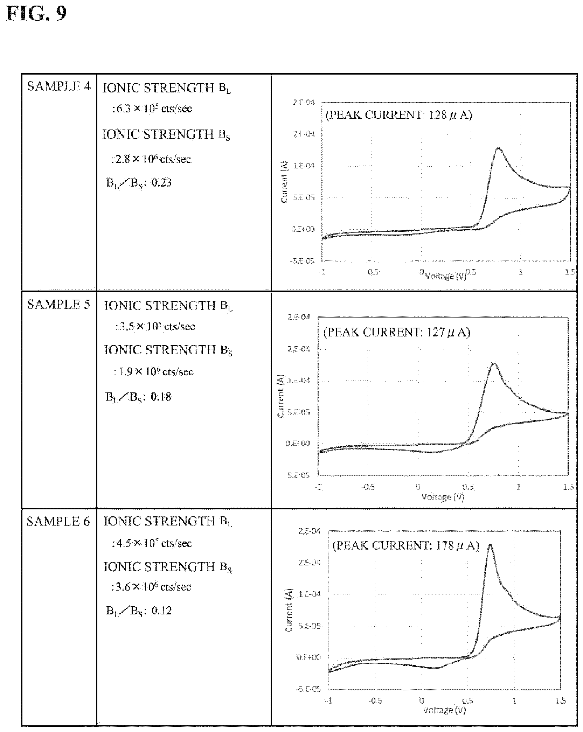

Using Samples 1 to 8, a cyclic voltammogram was obtained by measuring the uric acid concentration in the test liquid containing uric acid and protein using CV measurement. The cyclic voltammetry was performed under the same predetermined conditions, namely: within a voltage range including a range of -1 V or more to 1.5 V or less; and within a sweeping speed range of 0.1 V/s or more to 1 V/s or less. The obtained cyclic voltammograms are shown in FIG. 8 to FIG. 10. The "peak current" shown in FIG. 8 and FIG. 9 is the highest current value among the current values obtained by performing the CV measurement in each sample.

As can be seen from the TOF-SIMS results for Sample 1 shown in FIG. 6 and FIG. 7, for Sample 1 the peak intensities (signals) of ionic strength observed at a molecular weight of 100 or more, i.e. the peak intensities of ionic strength attributable to the polymer organic matter, are all 5 × 10³ cts/sec or less for both the TOF-SIMS evaluated for positive ions and the TOF-SIMS evaluated for negative ions. This indicates that the amount of polymer organic matter adhering to the exposed surface of the diamond film is small.

In addition, as can be seen from the TOF-SIMS results for Sample 1 shown in FIG. 6 and FIG. 7, for Sample 1 there are peaks where peak intensities (signal intensities) of ionic strength observed at a molecular weight of less than 100, i.e. peak intensities of ionic strength attributable to the low-molecular-weight organic matter, that are more than 5 × 10⁴ (5E4) cts/sec for both the TOF-SIMS evaluated for positive ions and the TOF-SIMS evaluated for negative ions. For example, for the TOF-SIMS (positive ions) for Sample 1 shown in FIG. 6, peak intensities exceeding 5 × 10⁴ cts/sec can be observed near the molecular weights of 29, 43, 55, and 69. This indicates that a certain amount or more of low-molecular-weight organic matter has adhered to the exposed surface of the diamond film.

It can be seen from FIG. 6 and FIG. 7 that the TOF-SIMS (positive ions) and the TOF-SIMS (negative ions) differ in the types of low-molecular-weight organic matter observed. In other words, it can be seen that the types of low-molecular-weight organic matter observed in the TOF-SIMS (negative ions) are greater in number than the types of low-molecular-weight organic matter observed in the TOF-SIMS (positive ions). For Sample 1, similarly to the TOF-SIMS (positive ions) results, the TOF-SIMS (negative ions) results with a larger number of types of low-molecular-weight organic matter observed have peaks where peak intensities of ionic strength attributable to low-molecular-weight organic matter are more than 5 × 10⁴ cts/sec, and all of the peak intensities attributable to polymer organic matter are 5 × 10³ cts/sec or less. In other words, in the TOF-SIMS (negative ions) results as well, the peak intensities attributable to the polymer organic matter are smaller by one order of magnitude or more than the peak intensities of the ionic strength attributable to the low-molecular-weight organic matter.

As described above, in Sample 1, the peak intensities attributable to the polymer organic matter are smaller by one order of magnitude or more than the peak intensities attributable to the low-molecular-weight organic matter. This indicates that the amount of polymer organic matter that reacts with (bonds to) the protein in the test liquid and forms the membrane on the exposed surface of the diamond film when the test liquid is in contact with the exposed surface of the diamond film is small.

Further, as can be seen from the TOF-SIMS results for Sample 8 shown in FIG. 6 and FIG. 7, for Sample 8 the peak intensities of ionic strength attributable to the polymer organic matter are higher than the peak intensities attributable to the polymer organic matter in Sample 1 for both the TOF-SIMS evaluated for positive ions and the TOF-SIMS evaluated for negative ions. For example, for the TOF-SIMS (positive ions) for Sample 8 shown in FIG. 6, peaks can be observed near the molecular weights of 107, 147, 207, 221, and 281. Thus, from FIG. 6 and FIG. 7, it can be seen that the integrated value B_{L} of ionic strength attributable to the polymer organic matter is clearly higher for Sample 8 than for Sample 1. This indicates that the amount of polymer organic matter adhering to the exposed surface of the diamond film is larger.

Further, as can be seen from the TOF-SIMS results for Sample 8 shown in FIG. 6 and FIG. 7, for Sample 8 the peak intensities attributable to the low-molecular-weight organic matter are somewhat higher than the peak intensities attributable to the low-molecular-weight organic matter in Sample 1 for both the TOF-SIMS evaluated for positive ions and the TOF-SIMS evaluated for negative ions. For example, for the TOF-SIMS (positive ions) for Sample 8 shown in FIG. 6, peaks can be observed near the molecular weights of 73 and 91 in addition to near the molecular weights of 29, 43, 55, and 69 where the peaks are observed for the TOF-SIMS (positive ions) for Sample 1. In other words, from FIG. 6 and FIG. 7, it can be seen that the integrated value Bs of ionic strength attributable to the low-molecular-weight organic matter is higher than the integrated value Bs for Sample 1. This is considered to be the effect of fragments occurring during the TOF-SIMS measurement. In other words, for Sample 8, the integrated value Bs is considered to be high due to factors other than the low-molecular-weight organic matter that is intentionally adhered to the exposed surface of the diamond film in order to enhance the liquid contact properties of the exposed surface of the diamond film. For Sample 8, it is considered that due to a large adherence amount of polymer organic matter on the exposed surface of the diamond film, the ionic strength attributable to the fragments occurring during the TOF-SIMS measurement increased, and as a result, the integrated value Bs increased.

It has been confirmed that, as with Sample 8, when the value (B_{L}/B_{S}) of the integrated value B_{L} relative to the integrated value Bs is greater than a predetermined value (e.g. more than 0.3), then when the test liquid comes into contact with the exposed surface of the diamond film, the protein membrane is formed on the exposed surface of the diamond film, which obstructs the detection target from reaching the exposed surface of the diamond film, and as a result, the sensor sensitivity decreases.

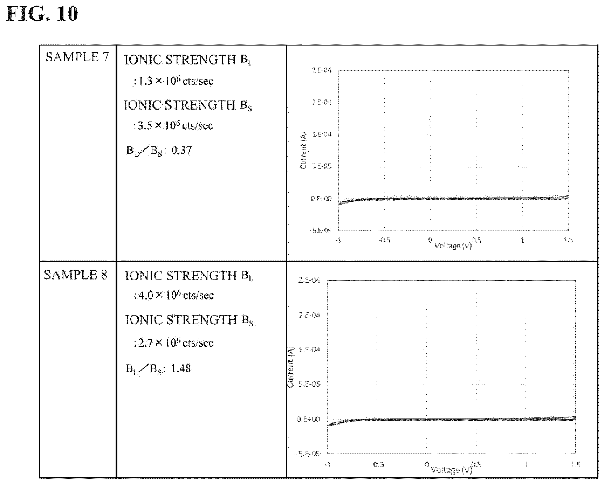

From FIG. 8 and FIG. 9, it can be seen that with Samples 1 to 6 for which the integrated value B_{L} is 1.2 × 10⁶ cts/sec or less, specifically, 6.3 × 10⁵ cts/sec or less, a peak current can be confirmed in the cyclic voltammogram and accurate CV measurement can be performed. That is, with Samples 1 to 6, it can be seen that a decrease in the uric acid concentration measurement accuracy is avoided. In contrast, from FIG. 10, it can be seen that with Samples 7 and 8 for which the integrated value B_{L} is more than 1.2 × 10⁶ cts/sec, a peak current cannot be confirmed in the cyclic voltammogram and accurate CV measurement cannot be performed. The reason therefor is considered to be that because the protein in the test liquid reacted with the polymer organic matter and formed a predetermined membrane on the exposed surface of the diamond film, as a result of which the detection target (uric acid) could not reach the exposed surface of the diamond film.

From FIG. 8 and FIG. 9, it can be seen that with Samples 1, 2, and 6 for which the integrated value B_{L} is 4.5 × 10⁵ cts/sec or less, the peak current is higher. The reason therefor is considered to be that because the adherence amount of polymer organic matter on the exposed surface of the diamond film is small, the formation of the aforementioned membrane on the exposed surface of the diamond film was reliably suppressed, and the amount of detection target reaching the exposed surface of the diamond film increased. In other words, from samples 1, 2, and 6, it can be seen that when the integrated value B_{L} at the exposed surface of the diamond film is 4.5 × 10⁵ cts/sec or less, a decrease in the accuracy of measuring the concentration of the detection target can be reliably avoided, and accurate concentration measurement can be reliably performed.

Moreover, from FIG. 8 and FIG. 9, it can be seen that when the integrated value Bs is 1.9 × 10⁶ cts/sec or more, the liquid contact properties of the exposed surface of the diamond film can be enhanced and accurate CV measurement can be reliably performed. For example, when comparing Sample 2 and Sample 5 with similar integrated values B_{L}, it can be seen that Sample 2 with a higher integrated value Bs has a higher peak current. The reason therefor is considered to be that because the adherence amount of low-molecular-weight organic matter on the exposed surface of the diamond film is large, the liquid contact properties were enhanced.

From FIG. 8 and FIG. 9, it can be seen that with Samples 2 and 6 for which the integrated value B_{L} is 4.5 × 10 ⁵ cts/sec or less and the integrated value Bs is 3.2 × 10⁶ cts/sec or more, the peak current is particularly high. The reason therefor is considered to be that because the formation of the aforementioned membrane on the exposed surface of the diamond film was reliably suppressed and the liquid contact properties could be reliably enhanced.

From FIG. 8 and FIG. 9, it can be seen that with Samples 1 to 6 for which the value of B_{L}/B_{S} is 0.3 or less, integrated value B_{L} is 1.2 × 10⁶ cts/sec or less and the integrated value Bs is 1.9 × 10⁶ cts/sec or more. From FIG. 10, it can be seen that with Samples 7 and 8 for which the value of B_{L}/B_{S} is more than 0.3, the integrated value B_{L} is more than 1.2 × 10⁶ cts/sec.

### <Preferable Aspects of the Present Disclosure>

Preferable aspects of the present disclosure will be supplementarily described hereafter.

### <Supplementary description 1>

According to an aspect of the present disclosure, there is provided an electrochemical sensor including:
a substrate;
an electrode chip including a base material and a diamond thin film; and
an insulating resin configured to seal at least part of a surface of a conductive member included in the electrode chip,
wherein the insulating resin is provided so as to expose at least part of the diamond thin film, and
when an exposed surface of the diamond thin film is measured by time-of-flight secondary ion mass spectrometry, an integrated value B_{L} per unit area of ionic strength of organic matter with a molecular weight of 100 or more is 1.2 × 10⁶ cts/sec or less, preferably 6.3 × 10⁵ cts/sec or less, more preferably 4.5 × 10⁵ cts/sec or less.

### <Supplementary description 2>

The sensor according to the Supplementary description 1,
wherein, preferably, when the exposed surface of the diamond thin film is measured by time-of-flight secondary ion mass spectrometry, an integrated value Bs per unit area of ionic strength of organic matter with a molecular weight of less than 100 is 1.9 × 10⁶ cts/sec or more, preferably 2.4 × 10⁶ cts/sec or more, more preferably 3.2 × 10⁶ cts/sec or more.

### <Supplementary description 3>

The sensor according to the Supplementary description 1 or 2,
wherein, preferably, a ratio (B_{L}/B_{S}) of the integrated value B_{L} to the integrated value Bs is 0.3 or less, preferably 0.23 or less.

### <Supplementary description 4>

The sensor according to any one of the Supplementary descriptions 1 to 3,
wherein, preferably, the exposed surface of the diamond thin film is oxygen-terminated.

### <Supplementary description 5>

The sensor according to any one of the Supplementary descriptions 1 to 4,
wherein, preferably, an oxide layer is provided on a surface of the insulating resin.

### <Supplementary description 6>

The sensor according to any one of the Supplementary descriptions 1 to 5,
wherein, preferably, the sensor is used in such a manner that a test liquid containing protein is in contact with the diamond thin film. In other words, preferably, a redox reaction is caused on the surface of the diamond thin film by applying a predetermined voltage in a state in which the test liquid containing protein is in contact with the diamond thin film.

### <Supplementary description 7>

The sensor according to any one of the Supplementary descriptions 1 to 6,
wherein, preferably, the sensor is used in such a manner that a test liquid containing bodily fluid, blood, or urine of an animal including human is in contact with the diamond thin film.

### DESCRIPTION OF SIGNS AND NUMERALS

10 Electrochemical sensor
11 Substrate
12 Electrode chip
121 Diamond (thin) film
121a Exposed surface of diamond film
122 Base material
19 Insulating resin

## Claims

1. An electrochemical sensor comprising:
a substrate;
an electrode chip including a base material and a diamond thin film; and
an insulating resin configured to seal at least part of a surface of a conductive member included in the electrode chip,
wherein the insulating resin is provided so as to expose at least part of the diamond thin film, and
when an exposed surface of the diamond thin film is measured by time-of-flight secondary ion mass spectrometry, an integrated value B_{L} per unit area of ionic strength of organic matter with a molecular weight of 100 or more is 1.2 × 10⁶ cts/sec or less.

2. The electrochemical sensor according to claim 1,
wherein when the exposed surface of the diamond thin film is measured by time-of-flight secondary ion mass spectrometry, an integrated value Bs per unit area of ionic strength of organic matter with a molecular weight of less than 100 is 1.9 × 10⁶ cts/sec or more.

3. The electrochemical sensor according to claim 2,
wherein a ratio of the integrated value B_{L} to the integrated value Bs is 0.3 or less.

4. The electrochemical sensor according to any one of claims 1 to 3,
wherein the exposed surface of the diamond thin film is oxygen-terminated.

5. The electrochemical sensor according to any one of claims 1 to 4,
wherein an oxide layer is provided on a surface of the insulating resin.

6. The electrochemical sensor according to any one of claims 1 to 5,
wherein the sensor is used in such a manner that a test liquid containing protein is in contact with the diamond thin film.

7. The electrochemical sensor according to any one of claims 1 to 6,
wherein the sensor is used in such a manner that a test liquid containing bodily fluid, blood, or urine of an animal including human is in contact with the diamond thin film.
